# FASCICULE DE BREVET EUROPEEN

(11) **EP 0 978 291 B1**
(45) Date de publication et mention de la délivrance du brevet: **01.10.2003**
(21) Numéro de dépôt: 99401804.2
(22) Date de dépôt: 19.07.1999
(51) Int. Cl.: A61M 16/12

(54) **Dispositif pour assistance respiratoire**
Vorrichtung zur Unterstützung der Atmung
Respiratory assistance device

(30) Priorité: 05.08.1998 FR 9810044
(43) Date de publication de la demande: 09.02.2000
(73) Titulaire: Boussignac, Georges, 92160 Antony (FR)
(72) Inventeur: Boussignac, Georges, 92160 Antony (FR)
(74) Mandataire: Bonnetat, Christian

(56) Documents cités:
- EP-A- 0 139 363
- EP-A- 0 701 834
- WO-A-95/28193
- US-A- 4 825 862

## Description

La présente invention a pour objet un dispositif pour assistance respiratoire, utilisable sur des patients dont la respiration spontanée est absente ou insuffisante, qu'ils soient placés ou non sous respiration artificielle.

On connaît divers dispositifs, tels que des masques, des sondes ou des canules orales, nasales, endotrachéales, trachéotomiques, destinés à faire la jonction entre un appareil de respiration artificielle et/ou d'anesthésie et le système respiratoire d'un patient. Ces dispositifs, essentiellement en forme de tubes, peuvent, selon le cas, comporter des moyens d'immobilisation tels que des pattes ou des collerettes au voisinage de l'extrémité proximale, pour le maintien sur la bouche ou le nez du patient, ou encore des ballonnets gonflables au voisinage de l'extrémité distale, pour le maintien par friction dans la trachée.

Les dispositifs connus présentent des inconvénients importants. Ainsi, par exemple, lorsqu'un tube de type connu est déconnecté du respirateur artificiel et que le patient a besoin d'air enrichi en oxygène, il est nécessaire d'introduire dans ledit tube une sonde reliée à une source d'oxygène. Par ailleurs, dans les cas de respiration spontanée insuffisante, le patient doit nécessairement rester relié au respirateur jusqu'au rétablissement complet de sa respiration spontanée.

Aussi, pour remédier à ces inconvénients, on a déjà proposé, par exemple dans les documents EP-A-0 390 684 et EP-A-0 701 834, des dispositifs d'assistance respiratoire qui, outre le canal principal formé par le tube, comportent au moins un canal auxiliaire, par exemple ménagé dans la paroi dudit tube, permettant l'injection d'un jet de gaz respirable (oxygène, air ou mélange air-oxygène) destiné à la ventilation du patient, ce canal auxiliaire débouchant dans le canal principal au voisinage de l'extrémité distale de ce dernier.

Bien entendu, ces derniers dispositifs d'assistance respiratoire connus comportent des moyens de sécurité, aptes à en arrêter le fonctionnement en cas de surpression dans le système respiratoire du patient et/ou dans ledit tube.

L'objet de la présente invention est de perfectionner lesdits dispositifs à assistance respiratoire pour en augmenter encore la sécurité d'utilisation.

A cette fin, selon l'invention, le dispositif d'assistance respiratoire comportant un tube qui forme un canal principal et qui est destiné à être relié par son extrémité distale à une voie respiratoire d'un patient pour que ledit canal principal relie, à l'extérieur, le système respiratoire dudit patient, ledit dispositif comportant de plus au moins un canal auxiliaire relié à une source de gaz respirable pour pouvoir insuffler un jet d'un tel gaz respirable dans ledit système respiratoire et débouchant dans ledit canal principal au voisinage de l'extrémité distale de ce dernier, est remarquable en ce que, dans la conduite reliant ladite source de gaz respirable audit canal auxiliaire, il comporte :
- du côté de ladite source, un dispositif à perte de charge apte à limiter le débit et la pression dudit gaz respirable disponible à la sortie de ladite source et à imposer audit jet de gaz respirable une valeur de débit et une valeur de pression prédéterminées ; et
- du côté dudit canal auxiliaire, une soupape d'échappement tarée apte à mettre ladite conduite en communication avec l'atmosphère ambiante, dès que la pression dans ladite conduite dépasse ladite valeur de pression prédéterminée.

Ainsi, grâce à la présente invention, la sécurité agit au niveau de l'injection du jet de gaz respirable et complète avantageusement les sécurités des dispositifs connus rappelés ci-dessus, sécurités qui agissent au niveau des voies respiratoires du patient.

On remarquera que, pour obtenir la sécurité maximale recherchée par la présente invention, l'association du dispositif à perte de charge et de la soupape d'échappement tarée est indispensable. En effet, le dispositif à perte de charge abaisse le débit et la pression du jet de gaz respirable, de sorte que la soupape d'échappement peut, lorsque cette pression dépasse ladite valeur prédéterminée, évacuer la totalité du jet de gaz respirable dans l'atmosphère. Dans le cas où ledit dispositif à perte de charge serait supprimé, le débit et la pression du jet de gaz respirable pourraient atteindre des valeurs telles que ledit jet, au moins en partie, pourrait traverser ladite soupape d'échappement tarée en direction des voies respiratoires du patient, au lieu d'être évacué dans l'atmosphère. Il pourrait alors en résulter de graves lésions pour le patient.

Un tel dispositif à perte de charge peut être de tout type connu, tel que restrictions à vis pointeau, canaux de faible diamètre interne, etc ... et il en est de même de ladite soupape d'échappement tarée, qui peut être du type à piston et cylindre percé, à bille ou clapet chargé par un ressort, etc ...

De préférence, ledit dispositif à perte de charge est réglable de façon à permettre d'imposer audit jet de gaz respirable une pluralité de valeurs de débit et de valeurs de pression prédéterminées. De même, il est avantageux que le tarage de ladite soupape d'échappement soit réglable. Ainsi, il est possible d'adapter le dispositif conforme à la présente invention au cas particulier de chaque patient.

Notamment dans le cas où au moins une partie de la conduite reliant la source de gaz respirable au canal auxiliaire est incorporée audit tube, le dispositif à perte de charge et/ou ladite soupape d'échappement tarée pourraient également être incorporés audit tube. Cependant, de préférence, ils sont, au contraire, extérieurs à celui-ci.

Grâce au dispositif à perte de charge, il est particulièrement aisé de prévoir un humidificateur dans ladite conduite reliant la source de gaz respirable au canal auxiliaire. En effet, ce dispositif à perte de charge permet d'abaisser la pression du jet de gaz respirable à un niveau permettant une bonne humidification de celui-ci. On évite ainsi le dessèchement de la muqueuse du patient. De préférence, ledit humidificateur est disposé entre le dispositif à perte de charge et la soupape d'échappement tarée.

Par ailleurs, pour éviter que le jet de gaz respirable humidifié vienne frapper directement la muqueuse, risquant par son énergie cinétique de traumatiser la muqueuse, il est avantageux que, comme cela a été décrit dans le brevet européen EP-A-0 390 684, au moins l'extrémité distale dudit canal auxiliaire débouchant dans le canal principal soit parallèle à celui-ci et que, en regard de l'orifice distal dudit canal auxiliaire, soient prévus des moyens de déflexion dudit jet de gaz respirable de ventilation vers l'intérieur dudit canal principal.

Ainsi, le jet de gaz respirable humidifié sous faible pression traversant ledit canal auxiliaire est défléchi vers l'axe du canal principal, lorsqu'il pénètre dans celui-ci. En aval desdits moyens de déflexion, c'est-à-dire à l'intérieur du canal principal, la pression dudit jet de gaz respirable chute et le jet sort à encore plus faible pression à travers l'orifice distal du tube. L'expérience a montré qu'en aval de la sortie distale du tube, la pression est faible et maintenue constante dans tout l'espace respiratoire. Cette pression est dépendante du débit de gaz respirable dans les canaux auxiliaires. Par suite, avec le dispositif d'assistance respiratoire conforme à l'invention, on peut par exemple apporter de l'oxygène humide ou un mélange air-oxygène humide directement dans les poumons, à hauteur de la carène, et supprimer ainsi l'espace mort qui existe dans les sondes actuelles et qui est d'environ un tiers du volume respiratoire total pour un adulte et environ la moitié pour les nouveau-nés prématurés.

La suppression de cet espace mort correspond à une augmentation de performance du cycle respiratoire de plus de 25% dans tous les cas de malades et de près de 50% dans certains cas.

Lorsque le dispositif selon l'invention comporte une pluralité de canaux auxiliaires, il est avantageux qu'au moins certains d'entre eux soient alimentés en commun en gaz respirable. Une telle alimentation en commun desdits canaux peut se faire par l'intermédiaire d'une bague de distribution, coaxiale audit tube. Par ailleurs, lesdits canaux auxiliaires non alimentés en commun peuvent servir à l'introduction de produits gazeux additionnels, tels que des produits médicamenteux.

Ainsi, on voit que le dispositif conforme à l'invention permet, en toute sécurité :
- l'humidification du gaz respirable insufflé,
- l'intubation de longue durée de l'assistance respiratoire sans assèchement,
- l'injection de médicaments ou d'anesthésiques pendant l'assistance respiratoire,
- la mesure dynamique des pressions, car il suffit de prévoir des canaux auxiliaires auxquels sont associées des sondes appropriées,
- l'établissement d'un microdébit de gaz respirable dans les canaux auxiliaires pour éviter l'obstruction desdits canaux par des mucosités,
- l'augmentation du volume échangé, car la pression est auto-limitée et il n'y a aucun risque d'écrasement des capillaires pulmonaires,
- la diminution pour une même quantité d'oxygène échangée de l'importance d'oxygène dans le mélange, ce qui diminue d'autant les effets secondaires de l'assistance,
- la possibilité d'utiliser des respirateurs moins coûteux que les respirateurs actuels.

Les figures du dessin annexé feront bien comprendre comment l'invention peut être réalisée. Sur ces figures, des références identiques désignent des éléments semblables.

La figure 1 est une vue schématique et partielle, en coupe axiale agrandie, d'un mode de réalisation du dispositif de l'invention.

Les figures 2 et 3 sont des coupes transversales, respectivement selon les lignes ll-ll et III-III de la figure 1.

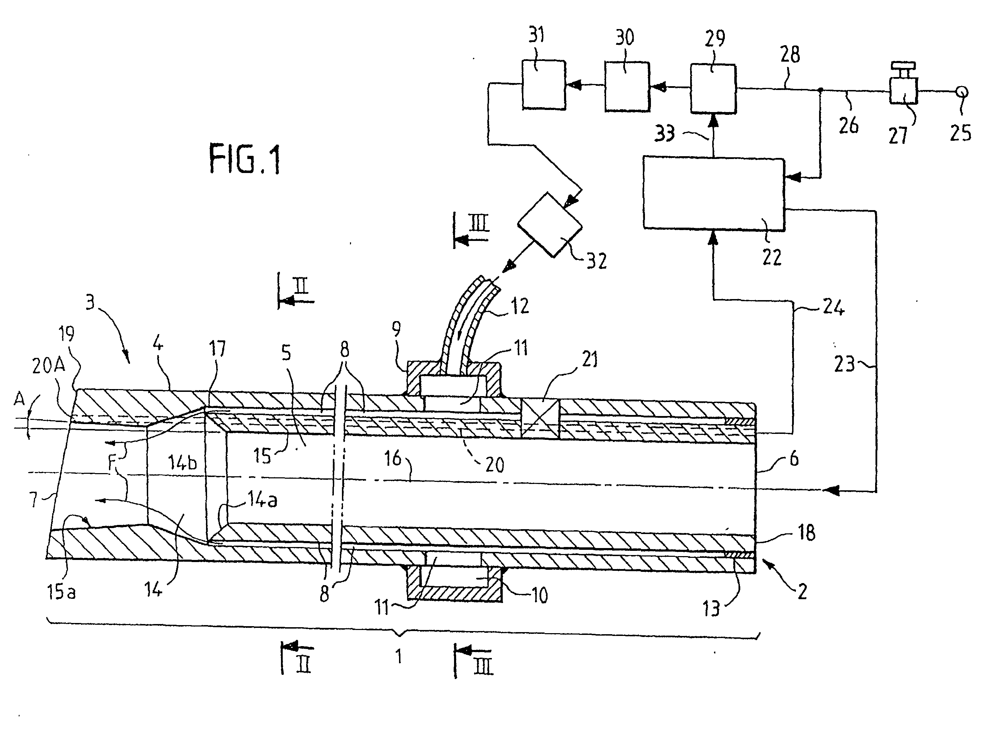

Sur la figure 1, on a représenté, schématiquement et à grande échelle, les seules extrémités proximale 2 et distale 3 d'un mode de réalisation 1 du dispositif selon l'invention. Ce mode de réalisation peut constituer, par exemple, une sonde endotrachéale oro-nasale avec ou sans ballonnet, une sonde endotrachéale pédiatrique, une sonde de monitorage des gaz, une sonde endobronchique, une sonde naso-pharyngée, une sonde d'intubation anatomique pour enfant, une sonde de Cole néonatale, une sonde canule de Gedel, une sonde nasale d'oxygénothérapie, un masque nasal ou bucconasal ou un ballon nasal pour traitement d'apnée du sommeil.

Le dispositif 1 comporte un tube 4, souple ou préformé (pour s'adapter à la morphologie du patient) délimitant un canal principal 5 débouchant, par l'orifice 6, à l'extrémité proximale 2 et, par l'orifice 7, à l'extrémité distale 3.

Ainsi, le canal principal 5 est capable d'assurer le passage entre les orifices 6 et 7, dont l'un (l'orifice 7) est destiné à se trouver à l'intérieur des voies respiratoires d'un patient, et l'autre (l'orifice 6) est destiné à se trouver à l'extérieur dudit patient. Cet orifice 6 peut déboucher à l'air libre et, dans ce cas, le patient peut inspirer de l'air frais et expirer l'air vicié à travers le canal principal 5. On peut également, comme cela est expliqué ci-après, relier l'orifice 6 à une source de gaz respirable sous pression et prévoir un système de valves unidirectionnelles, pour que le patient inspire le gaz respirable de ladite source à travers ledit canal principal 5 et expire le gaz vicié à l'air libre, également à travers ce canal principal.

Le diamètre du canal principal 5 est de l'ordre de quelques millimètres. Des essais satisfaisants ont été effectués avec des diamètres de 3 mm, 7 mm, 8 mm et 12 mm.

Par ailleurs, dans l'épaisseur de la paroi du tube 4, sont ménagés des canaux auxiliaires 8, s'étendant sur la presque totalité de la longueur du canal principal et destinés à être reliés à une source de gaz respirable sous pression, comme cela est décrit ci-après.

La liaison à la source de gaz respirable sous pression peut être réalisée au moyen d'une bague 9, entourant de façon étanche le tube 4, du côté de l'extrémité proximale 2, et délimitant une chambre annulaire étanche 10 autour dudit tube. Les canaux auxiliaires 8 sont mis en communication avec la chambre 10, grâce à des arrachements locaux 11 de la paroi du tube 4, et ladite chambre 10 est reliée à ladite source de gaz respirable par un conduit 12. Bien entendu, les extrémités proximales des canaux 8 sont obturées, par exemple par des bouchons 13, introduits à partir de la face d'extrémité proximale 18 du tube 4.

Les canaux auxiliaires 8 ont un diamètre plus petit que celui du canal principal 5. Le diamètre des canaux auxiliaires 8 est de préférence inférieur à 1 mm et, de façon avantageuse, il est de l'ordre de 400 à 800 microns. Du côté distal, les canaux auxiliaires 8 débouchent dans un évidement 14 de la paroi interne 15 du tube 4. L'évidement 14 est annulaire et centré sur l'axe 16 de l'extrémité distale 3. Il comporte une face 14a, sensiblement transversale ou légèrement inclinée de façon à constituer un évasement du canal principal 5, dans laquelle débouchent lesdits canaux auxiliaires 8 par leurs orifices 17, ainsi qu'une face 14b suivant la face 14a et convergeant en direction de l'axe 16.

De préférence, entre la face inclinée convergente 14b et l'orifice distal 7, la paroi interne 15 présente une partie 15a légèrement évasée vers l'extérieur, comme cela est illustré par l'angle A sur la figure 1.

Ainsi, lorsque les canaux auxiliaires 8 sont alimentés en gaz respirable sous pression à travers les éléments 9 à 12, les jets gazeux correspondants heurtent la face inclinée 14b, qui les défléchit en direction de l'axe 16 (flèches F sur la figure 1), engendrant au voisinage de celui-ci une zone de dépression favorisant la circulation gazeuse à l'intérieur du canal principal 5, de l'orifice proximal vers l'orifice distal. On favorise ainsi l'inspiration du patient.

De préférence, la distance entre chacun des orifices 17 et l'orifice 7 est de l'ordre de 1 à 2 cm.

Au moins un canal supplémentaire 20 est prévu dans l'épaisseur du tube 4 afin de déboucher en 20A au voisinage de l'extrémité distale 19 du tube 4 et servir de prise de pression.

A titre de sécurité, une soupape d'échappement tarée 21 peut être prévue dans l'extrémité proximale2 du tube 4. Ainsi, en cas de surpression accidentelle dans le canal principal 5, une fuite de gaz se produit à l'extérieur du patient, à travers la paroi du tube 4, pour éliminer instantanément cette surpression.

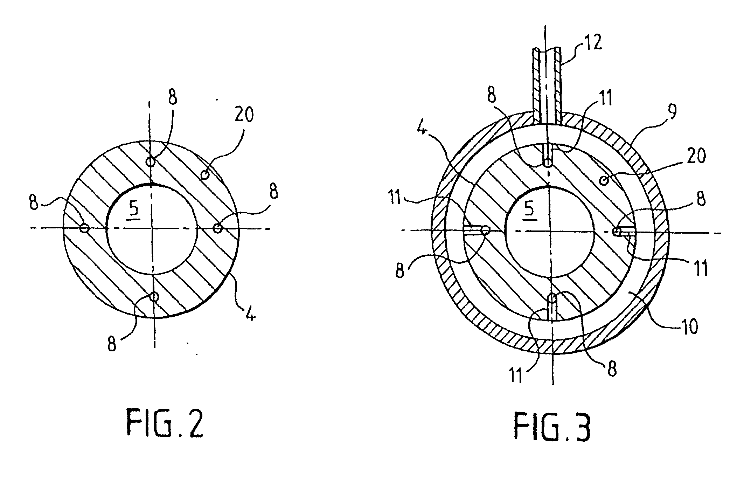

Comme le montrent les figures 2 et 3, les canaux auxiliaires 8 sont disposés régulièrement autour de l'axe du tube 4. Leur nombre est variable suivant les utilisations (adulte ou enfant), mais il est généralement compris entre trois et neuf. De plus, un au moins des canaux auxiliaires 8 peut être spécialisé pour apporter un fluide médical.

Le tube 4 du dispositif selon l'invention peut être réalisé en toute matière déjà utilisée dans les sondes respiratoires, par exemple en un chlorure de polyvinyle, avec un éventuel revêtement de silicone ou en acier permettant les injections à pression élevée.

Bien entendu, les dimensions du dispositif selon l'invention peuvent être très variables, essentiellement en fonction de la voie de mise en place du tube et de la taille du patient, qui peut être un adulte, un enfant, un nourrisson ou un prématuré.

Le dispositif 1 comporte de plus un dispositif d'alimentation et de commande 22, qui est respectivement relié à l'orifice 6 de l'extrémité proximale 2 du tube 4 par une liaison 23 et au canal supplémentaire 20 par une liaison 24.

Le dispositif d'alimentation et de commande 22 est alimenté en gaz respirable sous pression par une source 25, à laquelle il est relié par une conduite 26 sur laquelle est monté un détendeur-débitmètre réglable 27.

La sortie du détendeur-débitmètre 27 est reliée au conduit 12 par une conduite de dérivation 28 sur laquelle sont montés en série une vanne commandable 29, un dispositif à perte de charge réglable limiteur de débit et de pression 30, un humidificateur 31 et une soupape d'échappement tarée 32, à tarage réglable. La vanne commandable 29 est commandée par le dispositif d'alimentation et de commande 22 par l'intermédiaire d'une liaison 33.

A titre d'exemple non limitatif, le détendeur-débitmètre 27 peut délivrer, dans la conduite 28, le gaz respirable provenant de la source 25 sous une pression P, par exemple égale à 3,5 bars avec un débit maximal réglable de par exemple 32 litres par minute, alors que le limiteur de débit et de pression 30, recevant ce gaz respirable de la conduite 28, peut en abaisser la pression jusqu'à une valeur p, égale par exemple à 0,5 bar (pour un adulte) et à 0,07 bar (pour un enfant), et le débit jusqu'à une valeur d égale, par exemple, à 0,5 litre par minute. Quant à elle, la soupape d'échappement 32 est tarée à la pression p.

Les modes de fonctionnement du dispositif 1 selon l'invention sont les suivants :
- dans le mode de respiration artificielle, le dispositif d'alimentation et de commande 22, d'une part, commande la vanne 29 à la fermeture par l'intermédiaire de la liaison 33, de sorte que le conduit 12 n'est pas alimenté en gaz et, d'autre part, adresse du gaz respirable dans le tube 4 par l'intermédiaire de la liaison 23. Ce dispositif 22 comporte des moyens (non représentés) pour permettre le réglage de la pression et du débit de gaz respirable qu'il reçoit de la conduite 26 et qu'il adresse au tube 4. Si une surpression se produit dans la voie respiratoire du patient, elle est détectée et transmise, par le canal supplémentaire 20 et la liaison 24, au dispositif 22 qui arrête son fonctionnement. De plus, si cette surpression dépasse le seuil de tarage de la soupape tarée 21 --par exemple du fait que le canal supplémentaire 20 est obstrué par des mucosités et n'a pu transmettre l'information de surpression au dispositif 22-- cette soupape 21 s'ouvre et le canal principal 5 est mis à l'atmosphère ;
- dans le mode d'assistance respiratoire, le dispositif d'alimentation et de commande 22 coupe la liaison 23 pour mettre l'orifice 6 en communication avec l'atmosphère et commande la vanne 29 par la liaison 33 pour que celle-ci adresse au patient un jet, continuel ou impulsionnel, de gaz respirable à travers le limiteur 30, l'humidificateur 31, la soupape d'échappement tarée 32 et les canaux auxiliaires 8. Si une surpression se produit dans la voie respiratoire du patient, comme cela a été décrit ci-dessus, cette surpression est détectée et transmise par le canal supplémentaire 20, de sorte que le dispositif 22 ferme la vanne 29 et que la conduite 28 cesse d'adresser du gaz au patient. Si le canal supplémentaire 20 est obstrué, le dispositif 22 n'est pas averti de la surpression dans la voie respiratoire du patient et ne peut s'arrêter, mais cette surpression entraîne une augmentation de pression dans les canaux auxiliaires 8 et le conduit 12. Lorsque cette augmentation de pression atteint le seuil d'ouverture de la soupape de sécurité 32, celle-ci s'ouvre et le jet de gaz respirable n'est plus adressé au patient, mais au contraire est dérivé vers l'extérieur par ladite soupape de sécurité 32. Ainsi, bien que dans ce dernier cas la sécurité 20A, 20, 24, 22, 29 n'ait pu fonctionner, le jet de gaz respirable ne peut atteindre le système respiratoire du patient.

De ce qui précède, on voit donc que, grâce à l'invention, on obtient, avec une sécurité maximale, une assistance respiratoire humidifiée non agressive pour le patient, avec disparition quasiment totale de l'espace mort inhérent aux sondes connues.

## Revendications

1. Dispositif d'assistance respiratoire comportant un tube (4) qui forme un canal principal (5) et qui est destiné à être relié par son extrémité distale (3) à une voie respiratoire d'un patient pour que ledit canal principal (5) relie, à l'extérieur, le système respiratoire dudit patient, ledit tube comportant de plus au moins un canal auxiliaire (8) relié à une source de gaz respirable (25) pour pouvoir insuffler un jet d'un tel gaz respirable dans ledit système respiratoire et débouchant dans ledit canal principal (5) au voisinage de l'extrémité distale (7) de ce dernier,
**caractérisé en ce que**, dans la conduite (28) reliant ladite source de gaz respirable (25) audit canal auxiliaire (8), il comporte :
- du côté de ladite source (25), un dispositif à perte de charge (30) apte à limiter le débit et la pression dudit gaz respirable disponible à la sortie de ladite source (25) et à imposer audit jet de gaz respirable une valeur de débit et une valeur de pression prédéterminées ; et
- du côté dudit canal auxiliaire (8), une soupape d'échappement tarée (32) apte à mettre ladite conduite (28) en communication avec l'atmosphère, dès que la pression dans ladite conduite (28) dépasse ladite valeur de pression prédéterminée.

2. Dispositif selon la revendication 1, **caractérisé en ce que** ledit dispositif à perte de charge (30) est réglable de façon à permettre d'imposer audit jet de gaz respirable une pluralité de valeurs de débit et de valeurs de pression prédéterminées.

3. Dispositif selon l'une des revendications 1 ou 2, **caractérisé en ce que** le tarage de ladite soupape d'échappement tarée (32) est réglable.

4. Dispositif selon l'une quelconque des revendications 1 à 3, **caractérisé en ce que** ledit dispositif à perte de charge (30) est incorporé audit tube (4).

5. Dispositif selon l'une quelconque des revendications 1 à 3, **caractérisé en ce que** ledit dispositif à perte de charge (30) est extérieur audit tube (4).

6. Dispositif selon l'une quelconque des revendications 1 à 5, **caractérisé en ce que** ladite soupape d'échappement tarée (32) est incorporée audit tube (4).

7. Dispositif selon l'une quelconque des revendications 1 à 5, **caractérisé en ce que** ladite soupape d'échappement tarée (32) est extérieure audit tube (4).

8. Dispositif selon l'une quelconque des revendications 1 à 7, **caractérisé en ce qu'**il comporte un humidificateur (31) dans ladite conduite (28) reliant la source de gaz respirable (25) audit canal auxiliaire (8).

9. Dispositif selon la revendication 8, **caractérisé en ce que** ledit humidificateur (31) est disposé entre ledit dispositif à perte de charge (30) et ladite soupape d'échappement tarée (32.

10. Dispositif selon l'une des revendications 1 à 9, **caractérisé en ce qu'**au moins l'extrémité distale dudit canal auxiliaire (8) débouchant dans le canal principal (5) est parallèle à celui-ci et **en ce que**, en regard de l'orifice distal (17) dudit canal auxiliaire (8), sont prévus des moyens (14b) de déflexion dudit jet de gaz respirable de ventilation vers l'intérieur dudit canal principal (5).

## Patentansprüche

1. Vorrichtung zur Atmungsunterstützung, welche enthält: ein Rohr (4), welches einen Hauptkanal (5) bildet und welches dazu bestimmt ist, an seinem distalen Ende (3) mit einem Atemweg eines Patienten verbunden zu werden, damit der besagte Hauptkanal (5) die Verbindung vom Atmungssystem des besagten Patienten nach außen herstellt, wobei das besagte Rohr außerdem mindestens einen Hilfskanal (8) aufweist, der mit einer Quelle (25) für Atemgas verbunden ist, damit ein Strom eines solchen Atemgases in das besagte Atmungssystem eingeblasen werden kann, und der in den besagten Hauptkanal (5) in der Nähe des distalen Endes (7) dieses Letzteren einmündet,
**dadurch gekennzeichnet, dass** sie in der Leitung (28), welche die besagte Atemgasquelle (25) mit dem besagten Hilfskanal (8) verbindet, aufweist:
- auf der Seite der besagten Quelle (25) eine Drosselvorrichtung (30), die geeignet ist, den Volumenstrom und den Druck des am Ausgang der besagten Quelle (25) verfügbaren Atemgases zu begrenzen und dem Strom an Atemgas einen vorbestimmten Wert für den Volumenstrom und einen vorbestimmten Wert für den Druck aufzuprägen; und
- auf der Seite des besagten Hilfskanals (8) ein geeichtes Entlüftungsventil (32), das dazu geeignet ist, die besagte Leitung (28) mit der Umgebungsatmosphäre zu verbinden, sobald der Druck in der besagten Leitung (28) den besagten, vorbestimmten Druckwert übersteigt.

2. Vorrichtung nach Anspruch 1,
**dadurch gekennzeichnet, dass** die besagte Drosselvorrichtung (30) dergestalt einstellbar ist, dass ermöglicht wird, dem besagten Atemgasstrom eine Anzahl von vorbestimmten Werten für den Volumenstrom und den Druck aufzuprägen.

3. Vorrichtung nach einem der Ansprüche 1 oder 2, **dadurch gekennzeichnet dass** die Tarierung des besagten geeichten Entlüftungsventils (32) einstellbar ist.

4. Vorrichtung nach einem der Ansprüche 1 bis 3, **dadurch gekennzeichnet, dass** die besagte Drosselvorrichtung (30) in das besagte Rohr (4) eingebaut ist.

5. Vorrichtung nach einem der Ansprüche 1 bis 3, **dadurch gekennzeichnet, dass** sich die besagte Drosselvorrichtung (30) außerhalb des besagten Rohres (4) befindet.

6. Vorrichtung nach einem der Ansprüche 1 bis 5, **dadurch gekennzeichnet, dass** das besagte geeichte Entlüftungsventil (32) in das besagte Rohr (4) eingebaut ist.

7. Vorrichtung nach einem der Ansprüche 1 bis 5, **dadurch gekennzeichnet, dass** sich das besagte geeichte Entlüftungsventil (32) außerhalb des besagten Rohres (4) befindet.

8. Vorrichtung nach einem der Ansprüche 1 bis 7,
**dadurch gekennzeichnet, dass** sie einen Befeuchter (31) in der Leitung (28) enthält, welche die Quelle (25) für das Atemgas mit dem besagten Hilfskanal (8) verbindet.

9. Vorrichtung nach Anspruch 8,
**dadurch gekennzeichnet, dass** der besagte Befeuchter (31) zwischen der besagten Drosselvorrichtung (30) und dem besagten geeichten Entlüftungsventil (32) angeordnet ist.

10. Vorrichtung nach einem der Ansprüche 1 bis 9,
**dadurch gekennzeichnet, dass** mindestens das distale Ende des besagten Hilfskanals (8), das in den Hauptkanal (5) einmündet, zu diesem parallel ist, und dadurch, dass gegenüber der distalen Öffnung (17) des besagten Hilfskanals (8) Mittel (14b) zum Ablenken des besagten Stroms an Atemgas, der der Beatmung dient, zum Innern des besagten Hauptkanals (5) vorhanden sind.

## Claims

1. Device for respiratory assistance comprising a tube (4) which forms a main channel (5) and which is intended to be connected via its distal end (3) to the respiratory tract of a patient so that said main channel (5) connects the respiratory system of said patient to the outside, said tube moreover comprising at least one auxiliary channel (8) connected to a source of respirable gas (25) so as to permit the insufflation of a jet of such a respirable gas into said respiratory system, and opening into said main channel (5) in the vicinity of the distal end (7) of the latter, **characterized in that**, in the conduit (28) connecting said source of respirable gas (25) to said auxiliary channel (8), said device includes:
- toward said source (25), a device for loss of head (30) which is able to limit the flow rate and the pressure of said respirable gas available at the outlet of said source (25), and to impose on said jet of respirable gas a predetermined flow rate value and a predetermined pressure value; and
- toward said auxiliary channel (8), a calibrated exhaust valve (32) which is able to bring said conduit (28) into communication with the atmosphere when the pressure in said conduit (28) exceeds said predetermined pressure value.

2. Device according to Claim 1, **characterized in that** said device for loss of head (30) is adjustable in such a way as to be able to impose on said jet of respirable gas a plurality of predetermined flow rate values and pressure values.

3. Device according to either of Claims 1 and 2, **characterized in that** the calibration of said calibrated exhaust valve (32) is adjustable.

4. Device according to any one of Claims 1 and 2,
**characterized in that** said device for loss of head (30) is incorporated in said tube (4).

5. Device according to any one of Claims 1 to 3, **characterized in that** said device for loss of head (30) is external to said tube (4).

6. Device according to any one of Claims 1 to 5, **characterized in that** said calibrated exhaust valve (32) is incorporated in said tube (4).

7. Device according to any one of Claims 1 to 5,
**characterized in that** said calibrated exhaust valve (32) is external to said tube (4).

8. Device according to any one of Claims 1 to 7, **characterized in that** it comprises a humidifier (31) in said conduit (28) connecting the source of respirable gas (25) to said auxiliary channel (8).

9. Device according to Claim 8, **characterized in that** said humidifier (31) is arranged between said device for loss of head (30) and said calibrated exhaust valve (32).

10. Device according to any one of Claims 1 to 9, **characterized in that** at least the distal end of said auxiliary channel (8) opening into the main channel (5) is parallel thereto, and **in that** there are provided, opposite the distal orifice (17) of said auxiliary channel (8), means (14b) for deflecting said jet of respirable ventilation gas toward the inside of said main channel (5).
